# EUROPEAN PATENT APPLICATION

(11) **EP 4 817 557 A1**
(43) Date of publication of application: **07.10.2026**
(21) Application number: 24897622.7
(22) Date of filing: 28.11.2024
(51) Int. Cl.: A61C 13/00, A61C 5/70, A61C 8/00, A61K 6/20, A61K 6/802, A61K 6/818

(54) **METHOD FOR PRODUCING RECYCLED POWDER**

(30) Priority: 29.11.2023 JP 2023201429; 19.01.2024 JP 2024006646
(71) Applicant: Tosoh Corporation, Shunan-shi, Yamaguchi 746-8501 (JP)
(72) Inventor: TODOKO Shigehisa, Tokyo 104-8467 (JP); MACHIDA Kairi, Tokyo 104-8467 (JP); USHIO Yuki, Shunan-shi Yamaguchi 746-8501 (JP)
(74) Representative: Müller-Boré & Partner Patentanwälte PartG mbB
(86) International application number: PCT/JP2024/042078
(87) International publication number: WO 2025/115938

(57) **Abstract**

Provided is at least one of a recycled powder obtained by using horizontal recycling of scrap materials of dental blanks; a method for producing the recycled powder; a method for producing a recycled dental blank; and a recycled dental blank produced by the method. A method for producing a recycled powder includes a grinding step of grinding scrap materials of dental blanks to provide a ground product; and a collection step of collecting, from the ground product, a powder having a shade that has an L* of 90 or greater and 100 or less, an a* of 0 or greater and 1 or less and a b* of 0 or greater and 5 or less.

## Description

### Technical Field

The present disclosure relates to a method for producing a recycled powder, which is produced by subjecting dental blanks to a recycling process (recycling), and also relates to a method for producing a recycled powder, which is produced by subjecting dental blanks made of ceramic calcined bodies to recycling.

### Background Art

Sintered bodies (ceramic materials) made of alumina, zirconia or the like have excellent biocompatibility and are widely used as dental materials. Among ceramics, zirconia sintered bodies have aesthetic properties and high mechanical strength similar to those of natural teeth and are, therefore, frequently used in dental prostheses, such as crowns, bridges, inlays, onlays and abutments.

A method for preparing a dental prosthesis made of a sintered body is as follows. First, a raw material powder is molded to form a green body (green compact), and then the green body is calcined to prepare a calcined body (dental blank). Next, the calcined body is subjected to a milling process in a dental laboratory or the like to form the calcined body into a shape of a dental prosthesis, with the shape being configured taking into account thermal shrinkage caused by sintering. Subsequently, the calcined body is sintered to form a sintered body (dental prosthesis), which is then fine-tuned and eventually attached to a patient.

Incidentally, there is a growing demand to recycle ceramic materials in order to achieve a reduction in industrial waste, a reduction in environmental impact, and the like. There are known methods for recycling a ceramic material, and examples of the methods include a technique for recycling an electrolyte sheet for fuel cells made of a zirconia sintered body (Patent Literature 1) and a technique for recycling grinding balls made of a zirconia sintered body (Patent Literature 2).

### Citation List

### Patent Literature

PTL 1: Japanese Unexamined Patent Application Publication No. 2010-027358
PTL 2: Japanese Unexamined Patent Application Publication No. 10-218662

### Summary of Invention

### Technical Problem

It is believed that calcined bodies, including dental blanks, are suitable for recycling (in particular, horizontal recycling or closed-loop recycling), because they have not yet undergone a sintering process. Unfortunately, Patent Literature 1 and 2 both relate to a technique for recycling a sintered body, that is, they do not relate to the technique for recycling a dental blank, in particular, recycling scrap materials of dental blanks resulting from the milling process.

Furthermore, regarding the dental blank, conforming to the properties of a desired dental prosthesis requires using a plurality of types of dental blanks. Scrap materials of dental blanks resulting from the milling process are a mixture of materials of different compositions and types, and, therefore, collecting such scrap materials for the purpose of recycling is difficult in itself. For this reason, currently, such scrap materials are disposed of as waste by the process operator, such as a dental laboratory, or at best, such scrap materials may be used in cascade recycling, for example, by repurposing them as raw materials for cement. Even if the scrap materials of dental blanks are successfully collected, it is essential, in the case of horizontal recycling, that composition analysis be performed on each of the collected scrap materials before the scrap materials are separated. Thus, horizontal recycling of scrap materials of dental blanks has been practically impossible.

An object of the present disclosure is to provide at least one of a recycled powder obtained by using horizontal recycling of scrap materials of dental blanks; a method for producing the recycled powder; a method for producing a recycled dental blank; and a recycled dental blank produced by the method.

### Solution to Problem

In the present disclosure, studies were conducted on the feasibility of the recycling, particularly, horizontal recycling, of dental blanks, by focusing on the shade of the scrap materials of dental blanks resulting from the milling process. As a result, the following discoveries were made. The recycling process (recycling) of the scrap materials of dental blanks discarded during a production process for dental prostheses can be accomplished by performing a simple operation on the scrap materials, which makes it possible for the scrap materials to serve as a raw material for a dental blank. Furthermore, a powder obtained by using such a recycling process is suitable for use as a raw material for dental blanks.

Specifically, the present invention is as described in the claims, and a summary of the present disclosure is as follows.
[1] A method for producing a recycled powder, the method comprising a grinding step of grinding scrap materials of dental blanks to provide a ground product; and a collection step of collecting, from the ground product, a powder having a shade that has an L* of 90 or greater and 100 or less, an a* of 0 or greater and 1 or less and a b* of 0 or greater and 5 or less.
[2] The method for producing a recycled powder according to [1], further comprising a separation step of separating the scrap materials prior to the grinding step.
[3] The method for producing a recycled powder according to [2], wherein the separation step includes separating the scrap materials into a first shade unit and a second shade unit, the first shade unit is formed of at least one scrap material having a shade that has an L* of 90 or greater and 100 or less, an a* of 0 or greater and 1 or less and a b* of 0 or greater and 5 or less, and the second shade unit is formed of at least one scrap material having a shade different from the shade of the first shade unit.
[4] The method for producing a recycled powder according to any one of [1] to [3], wherein the grinding step includes a coarse grinding step and a particle size adjustment step, the coarse grinding step is a step of dry-grinding the scrap materials to provide a coarsely ground product, and the particle size adjustment step is a step of wet-grinding the coarsely ground product.
[5] The method for producing a recycled powder according to any one of [1] to [4], wherein the collection step includes separating the ground product provided in the grinding step into several units and then collecting, from the several units, a unit corresponding to a portion of the ground product, the portion having a shade that has an L* of 90 or greater and 100 or less, an a* of 0 or greater and 1 or less and a b* of 0 or greater and 5 or less.
[6] A method for recycling dental blanks, the method comprising a grinding step of grinding scrap materials of the dental blanks to provide a ground product; and a collection step of collecting, from the ground product, a powder having a shade that has an L* of 90 or greater and 100 or less, an a* of 0 or greater and 1 or less and a b* of 0 or greater and 5 or less.
[7] A method for producing a recycled dental blank, the method comprising using the recycled powder obtainable according to at least one of [1] to [5].
[8] A recycled powder having a shade that has an L* of 90 or greater and 100 or less, an a* of 0 or greater and 1 or less and a b* of 0 or greater and 5 or less.

### Advantageous Effects of Invention

The present disclosure can provide at least one of a recycled powder obtained by using horizontal recycling of scrap materials of dental blanks; a method for producing the recycled powder; a method for producing a recycled dental blank; and a recycled dental blank produced by the method.

### Brief Description of Drawings

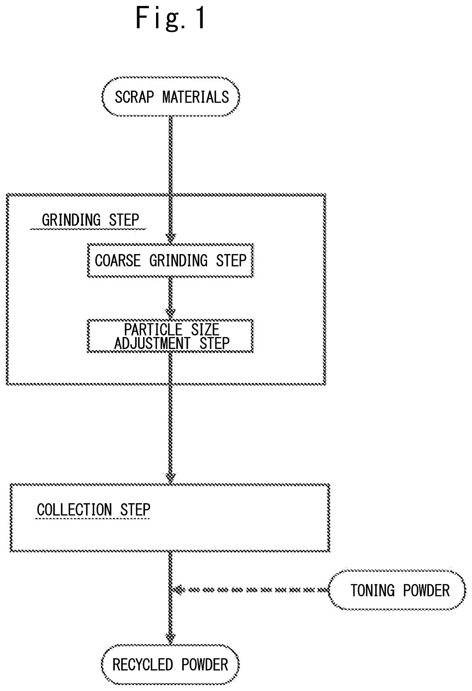

[Fig. 1] A flowchart of a method for producing a recycled powder that includes no separation step.

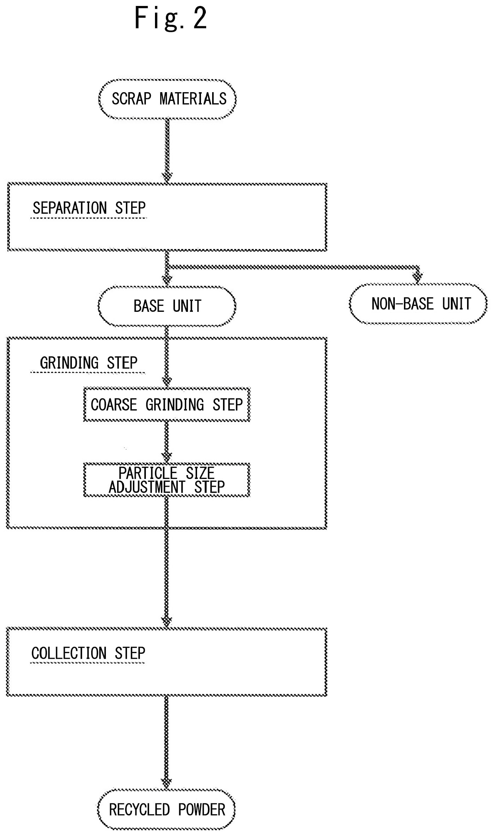

[Fig. 2] A flowchart of a method for producing a recycled powder that includes a separation step.

### Description of Embodiments

The present disclosure will be described below with reference to examples of embodiments. Any combination of any of the features and numerical values disclosed in this specification is encompassed by the present disclosure. In addition, ranges defined by any combination of any of the upper limits and lower limits of the numerical values disclosed in this specification are also encompassed by the present disclosure. Key terms in the present disclosure are described below.

A "dental prosthesis" is at least one of a crown, a bridge, an inlay, an onlay, an abutment, other dentures and other dental coatings. In particular, the dental prosthesis is at least one of dentures and dental coatings made of a ceramic material (sintered body).

A "dental blank" is a composite that serves as a precursor of a dental prosthesis. In particular, the dental blank is a ceramic calcined body suitable as a precursor of a dental prosthesis. Sintering a dental blank yields a dental prosthesis.

A "scrap material of a dental blank" (hereinafter also simply referred to as a "scrap material") is the remaining portion of a dental blank from which a shape of a dental prosthesis has been milled by a milling process, such as a CAD/CAM process, and the scrap material is at least one of a dental blank having one or more holes (milled holes) corresponding to a shape of a dental prosthesis, and fragments of such a dental blank.

A "recycled dental blank" (hereinafter also referred to as a "recycled blank") is a dental blank produced by using a recycled powder (described later) as a part or the whole of the raw material.

A "sintered body" is a composite made of ceramic grains and having a specific shape, and the composite can be prepared by molding and sintering a ceramic powder (the molding may be followed by calcining, if necessary).

A "calcined body" is a composite made of chemically adhered ceramic particles and having a specific shape, and the composite can be prepared by calcining (pre-sintering or partially sintering) a green body (green compact) prepared by molding a ceramic powder.

A "recycled powder" is a powder obtained by subjecting a sintered body, a calcined body or a green body to a recycling process (recycling) or is a mixed powder containing such a powder. In the present embodiment, in particular, the recycled powder is a powder obtained by subjecting a calcined body to a recycling process or is a mixed powder containing such a powder.

A "synthetic powder" is a powder other than the powder obtained by subjecting a sintered body, a calcined body or a green body to a recycling process. In particular, the synthetic powder is a powder prepared by one or more methods selected from the group of a hydrothermal synthesis method, a hydrolysis method and a neutralization co-precipitation method or by a different liquid phase method.

A "shade" is a shade measured with a spectrophotometer (e.g., CM-700d, manufactured by Konica Minolta Inc.) equipped with illumination and detection optics in accordance with Geometric condition c of JIS Z 8722; the measurement is performed with a black background in the SCI mode, and the shade is represented in the L*a*b* color system. In the case of a composite having flowability, such as powders, the shade of the composite is determined by performing the measurement on a green body (green compact) molded from the powder. The shade represented in the L*a*b* color system is a shade corresponding to the coordinates of the color space defined by three values, namely, a lightness L*, a chromaticity a* and a chromaticity b*.

### [Method for Producing Recycled Powder]

The present embodiment is a method for producing a recycled powder comprising a grinding step and a collection step. The grinding step is a step of grinding scrap materials of dental blanks to provide a ground product. The collection step is a step of collecting, from the ground product, a powder having a shade that has an L* of 90 or greater and 100 or less, an a* of 0 or greater and 1 or less and a b* of 0 or greater and 5 or less. The production of dental prostheses involves the use of a wide variety of dental blanks, and scrap materials of these are collectively discarded (disposed of). Thus, the discarded scrap materials (a gathering of scrap materials) are significantly different from one another in properties, including the shade, a chemical composition and constituent elements. Under these circumstances, the production method of the present embodiment is capable of collecting a powder that has a specific shade from those scrap materials (a gathering of scrap materials). The collected powder can be recycled to form a recycled powder suitable as a raw material powder for dental blanks.

Each of the steps of the method for producing a recycled powder of the present embodiment will be described below with reference to an example in which the scrap materials of dental blanks are scrap materials of zirconia calcined bodies.

### <Grinding Step>

The grinding step, in which scrap materials of dental blanks are ground to provide a ground product, is a step of grinding scrap materials to form a ground product. The ground product is a product formed from the scrap materials and being in a powder form, and the ground product itself can be regarded as a recycled powder. However, the scrap materials that are subjected to the grinding step may include a large number of scrap materials that differ in properties. Thus, the collection step, which is described below, is to be performed so that the resulting ground product can be a recycled powder more suitable as a raw material powder for dental blanks.

The scrap materials that are subjected to the grinding step are calcined bodies, ceramic calcined bodies or calcined bodies of zirconia (zirconium dioxide, ZrO₂) (hereinafter also referred to as "zirconia calcined bodies"). The calcined bodies are formed of particles that are chemically adhered to one another at an early stage of sintering, and the chemically adhered particles are particles in a state in which the particles are necked together. Compared with sintered bodies, in which grains are strongly bonded to one another via grain boundaries, calcined bodies are suitable for recycling processes involving powdering, because the energy necessary for grinding calcined bodies is very low.

The zirconia calcined body may be a calcined body consisting of zirconia or may be one that contains zirconia as a major component and contains, as minor components, metal elements, such as a stabilizing element and a coloring element.

The stabilizing element may be one or more selected from the group of yttrium (Y), calcium (Ca) and magnesium (Mg). The coloring element may be one or more selected from the group of titanium (Ti), iron (Fe), cobalt (Co), nickel (Ni), manganese (Mn), praseodymium (Pr), neodymium (Nd), europium (Eu), gadolinium (Gd), terbium (Tb), erbium (Er) and ytterbium (Yb). Another metal element may be one or more selected from the group of aluminum (Al), silicon (Si) and germanium (Ge). These metal elements may exist in any form; they may be dissolved in zirconia or may exist as oxides or other compounds.

The scrap materials may have a structure formed of one layer, that is, a structure having an entire composition. Meanwhile, dental prostheses need to be in aesthetic harmony with the surrounding tooth structure (natural teeth of the patient). In order to provide aesthetic properties similar to the aesthetic properties of the natural teeth, in which the aesthetic properties change over the area from the tooth root to the prepared surface, the scrap materials may have a structure formed of two or more layers (e.g., 2 or more and 15 or fewer layers, 2 or more and 10 or fewer layers or 3 or more and 8 or fewer layers). In these cases, the structure of the scrap materials may be one in which the layers are disposed such that the boundaries between adjacent layers are distinct, or alternatively, the structure may be one in which two or more layers are disposed such that a gradient is formed. The gradation may be any gradation that is formed by at least one of shades and translucencies. In the case where the dental blank has a structure in which two or more layers are disposed, it is sufficient that the metal element such as a coloring element be included in at least one of the layers, and it is preferable that the layers differ from one another in at least one of the type and the content of the coloring element.

In the grinding step, the grinding may be any grinding that enables the preparation of a ground product that can be used as a recycled powder of the scrap materials. Such grinding may be grinding that enables the ground product of the scrap materials to have an average particle size of 0.1 µm or greater, 0.3 µm or greater or 0.4 µm or greater and 10 µm or less, 5 µm or less or 1 µm or less. Preferably, the grinding is grinding that enables the ground product of the scrap materials to have an average particle size of 0.1 µm or greater and 10 µm or less or 0.4 µm or greater and 1 µm or less.

The average particle size of the ground product of the scrap materials can be determined by a Planimetric method that uses an SEM image. Specifically, a circle having a known area is drawn on an SEM image, and the number of particles (Nc) of the ground product within the circle and the number of particles (Ni) of the ground product on the circumference of the circle are measured. The average particle size of the ground product can be determined using equation (1), shown below, on the premise that the total number of particles (Nc+Ni) is 250 ± 50. 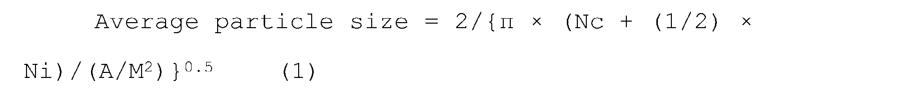 Average particle size = 2/{π × (Nc + (1/2) × Ni)/(A/M2)}0.5 (1)

In equation (1), Nc is the number of particles of the ground product within the circle, Ni is the number of particles of the ground product on the circumference of the circle, A is the area of the circle, and M is the magnification for the SEM observation (e.g., 5000 to 10000×). If the number of particles (Nc+Ni) in one SEM image is less than 200, two or more SEM images can be used to obtain an (Nc+Ni) of 250 ± 50.

Regarding grinding methods, using at least one of dry grinding and wet grinding is sufficient, that is, either of dry grinding and wet grinding can be used. The dry grinding may be, for example, a grinding method that uses one or more selected from the group of a jaw crusher, a hammer crusher, a shredder, a roll crusher, a hammer mill, a cutting mill, a rod mill, a roller mill, a rotor mill, an impact mill, a jet mill, a ball mill and a mortar. The wet grinding may be, for example, a grinding method that uses one or more selected from the group of a ball mill, a bead mill, a planetary mill, a wet jet mill, a colloid mill and a homogenizer. A preferred grinding method is wet grinding that uses, as the grinding medium, zirconia spheres (at least one of zirconia balls and zirconia beads). In this case, impurity contamination due to grinding is unlikely to occur.

Preferably, the grinding step is a grinding step including a coarse grinding step and a particle size adjustment step, the coarse grinding step is a step of dry-grinding the scrap materials to provide a coarsely ground product, and the particle size adjustment step is a step of wet-grinding the coarsely ground product. The coarse grinding step grinds the scrap materials into a coarsely ground product of the order of millimeters, for example, a coarsely ground product of 1 to 10 mm. With this coarse grinding step, it is possible to more efficiently achieve the above-mentioned average particle size of the ground product of the scrap materials, and, consequently, in the grinding step, the grinding time can be shortened, and the energy necessary for the grinding can be reduced.

### <Collection Step>

The production method of the present embodiment includes the collection step, in which a powder having a shade that has an L* of 90 or greater and 100 or less, an a* of 0 or greater and 1 or less and a b* of 0 or greater and 5 or less, is collected from the coarsely ground product. The powder having a shade that has an L* of 90 or greater and 100 or less, an a* of 0 or greater and 1 or less and a b* of 0 or greater and 5 or less (hereinafter this powder is also referred to as a "base powder", and this shade is referred to as a "base shade") is a powder obtained from scrap materials. The base powder is made of the same components as the dental blanks, which are the precursor of a dental prosthesis. Accordingly, the base powder is suitable for horizontal recycling for dental blanks. Furthermore, the base powder has a shade similar to the shade of a dental blank from which a dental prosthesis having the lightest shade, according to a shade guide (e.g., the Vita Classical shade guide), can be obtained.

The base powder can directly serve as a raw material powder for dental blanks. In addition, a powder (hereinafter also referred to as a "toning powder") having a different shade from that of the base powder may be mixed with the base powder to form a mixed powder. In this case, the mixed powder can serve as a raw material powder for dental blanks suitable for a dental prosthesis having a shade different from the base shade.

It is sufficient that the shade of the base powder has the base shade; preferably, the base shade has a lightness L*, a chromaticity a* and a chromaticity b* that satisfy the following values. The lightness L*, the chromaticity a* and the chromaticity b* indicate a single shade corresponding to the coordinates represented by the three values, and they do not independently represent respective shades.

Lightness L*: 90 or greater or 95 or greater and 100 or less or 99 or less,
Chromaticity a*: 0 or greater or 0.2 or greater and 1 or less or 0.8 or less, and
Chromaticity b*: 0 or greater or 0.2 or greater and 5 or less, 3 or less or 0.8 or less

The toning powder may be any powder having a shade that has an L* of less than 90, an a* of less than 0 or greater than 1 or a b* of less than 0 or greater than 5 and containing components similar to those of the base powder described above. The toning powder may be at least one of a powder consisting solely of zirconia and a zirconia powder containing a stabilizing element. Specifically, the toning powder may be at least one of a synthetic powder and a powder that is a ground product of scrap materials and has a shade different from that of the base powder.

Typically, dental blanks contain a coloring element. The scrap materials that are collected have different contents of the coloring element and are a gathering of dental blank residues resulting from the milling process. As such, the shade of the scrap materials is darker than the base shade. In this case, the shade of the ground product provided in the grinding step is also darker than the base shade, and, accordingly, it is preferable that a powder having a lighter shade, such as a powder of an yttrium-stabilized zirconium, be mixed as a toning powder.

In the collection step, any method for collection may be used as long as the base powder can be collected. For example, the method for collection may be one in which the ground product provided in the grinding step is separated into several units, from which a unit corresponding to a portion of the ground product, which is a portion having the base shade, is collected. A similar operation may be performed on the coarsely ground product provided in the coarse grinding step to separate the coarsely ground product into units, and, of these units, a unit corresponding to the base powder may be subjected to the particle size adjustment step, which may be followed by solid-liquid separation and drying; accordingly, the unit can be collected. The "unit" mentioned herein may be any unit that is at least one of a mass unit and a volume unit and can serve as a lot suitable for other processes and equipment of the recycling process.

The base powder that is collected can be recycled to serve as a recycled powder or a raw material for various ceramic materials. In particular, the base powder can be used as a powder suitable as a raw material for dental blanks.

### <Separation Step>

Preferably, the production method of the present embodiment includes a separation step of separating the scrap materials prior to the grinding step. The scrap materials are dental blanks resulting from a milling process. The scrap materials are a gathering of used dental blanks having different respective shades and compositions and are collected from process operators, such as dental laboratories. In the case where the separation step is included, a property of the scrap materials that are subjected to the grinding step, or, in other words, the shade of the ground product resulting from the grinding step, can be readily estimated. Consequently, the separation into units in the collection step or the coarse grinding step is simplified or made unnecessary, and, therefore, more efficient collection of the base powder can be anticipated.

The separation step separates the scrap materials. The separation may be carried out by any method that separates the scrap materials into units based on a specific property. The method is preferably one that separates the scrap materials into units according to at least one of the composition and the shade and more preferably one that separates the scrap materials into units according to the shade.

For example, in the case where the scrap materials are separated according to the shade, the method may be any method that separates the scrap materials into a first shade unit and a second shade unit. The first shade unit (hereinafter also referred to as a "base unit") is formed of at least one scrap material having a shade that has an L* of 90 or greater and 100 or less, an a* of 0 or greater and 1 or less and a b* of 0 or greater and 5 or less. The second shade unit (hereinafter also referred to as a "non-base unit") is formed of at least one scrap material having a shade different from the base shade. In the case where the scrap materials are separated according to the composition, the method may be any method that separates the scrap materials into a first composition unit and a second composition unit. The first composition unit is formed of at least one scrap material having a composition that provides the base shade. The second composition unit is formed of at least one scrap material having a composition that provides a shade different from the base shade. The analysis of the composition requires the taking of a sample, analysis on equipment and the like for each of the scrap materials, whereas the analysis of the shade can be performed more simply by non-contact optical analysis (e.g., during conveyance on a conveyor belt). Accordingly, regarding the method for separation, the separation using the shade-based units (shade units) is more preferable than the separation using the composition-based units (composition units).

The scrap materials that have been separated into the non-base unit can be subjected to the grinding process and the collection process described above to be recycled, as with the base powder, to serve as a toning powder or a raw material for other ceramic materials.

The non-base unit may be divided into two or more sub-units. In this case, the shade and the composition of the scrap material in each of the sub-units are more segmented and uniform, and, therefore, the scrap materials become more suitable for recycling in terms of shade control, which is achieved by being mixed with the base powder, and other applications.

Preferably, the scrap materials are each a scrap material of a dental blank provided with an information tag containing at least identification information associated with material information, so that the separation of scrap materials in the separation step can be facilitated. The "identification information" is information that can be associated with material information, and the "material information" preferably contains one or more pieces of information selected from the group of composition information, product information and production information (each will be described below).

Preferably, the identification information contains one or more selected from the group of raw material ratios, the composition (chemical composition), an average composition, the constituent elements, the shade, a shade code, an average shade and an average shade code (hereinafter, these pieces of information are also referred to as "composition information"). The composition information enables the properties of the scrap materials to be directly ascertained. It is preferable that the identification information contains one or more selected from the group of the raw material ratios, the composition, the constituent elements, the shade and the shade code, or it is preferable that the identification information contains at least one of the composition and the shade code. In the case where a scrap material has a structure formed of two or more layers, it is preferable that the composition information contain one or more selected from the group of the average composition, the average shade and the average shade code, in addition to the composition, the shade and the shade code, or in place of the composition, the shade and the shade code. The "raw material ratios" are information related to the types (e.g., the product names of the powders) and the amounts of use of the various raw materials used for the production of a dental blank. The shade is the desired shade of the dental prosthesis that is obtained by sintering the scrap materials (dental blanks). The "shade code" is a code indicating each of the shades in a shade guide, which is, for example, the code represented by any of the letters and numbers, ranging from A1 to D4, of the Vita Classical shade guide.

Preferably, the identification information contains at least one of the model number and the name of the manufacturer of the dental blanks (scrap materials) (hereinafter, these pieces of information are also referred to as "product information"), or preferably, the identification information contains at least the name of the manufacturer. The "model number" is the product name of a dental blank for the manufacturer of the dental blank. In the case where the identification information contains product information, rough separation after collection is made possible. Specifically, the scrap materials collected from process operators, such as dental laboratories, may include products of different manufacturers of dental blanks. In such a case, separation for each manufacturer or separation for each manufacturer and product is made possible, which facilitates efficient separation for horizontal recycling.

Preferably, the identification information contains one or more selected from the group of a production lot, a place of production, a year of production and a collection deadline of the dental blanks (hereinafter, these pieces of information are also referred to as "production information"). The identification information more preferably contains at least one of the year of production and the collection deadline and even more preferably contains the collection deadline. In the case where the production information is included, not only recycling is made possible, but also traceability is made possible. The "year of production" is information from which the time of production can be identified. The year of production may be replaced by a month and year of production or a date of production.

Preferably, the identification information is provided by an information tag including a code symbol, such as a code tag. Regarding the information tag, the code symbol may be read by a code symbol reading means, which is specifically, for example, at least one of a bar code reader and image analysis. In this case, the separation of scrap materials can be facilitated. The information tag may contain one or more selected from the group of letters, numbers and symbols. The type, size, format and the like of the code symbol may be appropriately selected in accordance with, for example, the amount of information provided to the information tag or a shape of the dental blanks. For example, the code symbol may be any code symbol that is at least one of a one-dimensional code symbol and a two-dimensional code symbol, specifically, at least one of a bar code and a QR code (registered trademark). The information tag may contain a unique code and the like. Preferably, the code symbol is one from which information can be read even when the code symbol is partially damaged.

### <Cleaning Step>

An impurity-reducing step for reducing impurities may be included; this step may precede or follow any of the above-described steps, namely, the separation step, the grinding step, the coarse grinding step, the particle size adjustment step and the collection step. This step can reduce impurities incidentally present in the collected scrap materials and impurities that are unintentionally incorporated before or after any of the steps.

Regarding methods for reducing impurities, any method that reduces impurities in the ground product or recycled powder (hereinafter also referred to as "scrap materials or the like") may be used, and the method may be cleaning or water washing.

In the case where, for example, water washing is used to reduce impurities, a drying step for drying the water-washed scrap materials or the like may be performed after the impurity-reducing step. Regarding methods for the drying, any method that reduces water that has been physically adsorbed onto the scrap materials or the like may be used. For example, the method may include performing drying in an air atmosphere at a temperature of 80°C or greater and 150°C or less. A duration of the drying may be appropriately adjusted in accordance with the amount of the scrap materials or the like that are dried and the features of a dryer. For example, the duration may be 30 minutes or more and 24 hours or less.

Preferably, the production method of the present embodiment includes the impurity-reducing step as a first step. Preferably, the impurity-reducing step precedes the grinding step (or the separation step, in the case where the separation step is included). The impurity-reducing step may be an independent step or may be performed during the transfer of the scrap materials or the like to any of the subsequent steps.

### [Method for Recycling Scrap Materials of Dental Blanks]

The method for producing a recycled powder of the present embodiment can be regarded as a method for recycling scrap materials of dental blanks (hereinafter also referred to as a "method for recycling of the present embodiment") including the grinding step of grinding scrap materials of dental blanks to provide a ground product and a collection step of collecting, from the ground product, a powder having a shade that has an L* of 90 or greater and 100 or less, an a* of 0 or greater and 1 or less and a b* of 0 or greater and 5 or less. As stated above, the recycled powder can serve as a raw material powder for dental blanks, that is, horizontal recycling of scrap materials can be achieved.

In the method for recycling of the present embodiment, the grinding step and the collection step are similar to those of the above-described method for producing a recycled powder, and in addition, at least one of a separation step and an impurity-reducing step may be included as in the above-described method for producing a recycled powder.

### [Recycled Powder]

The recycled powder produced by the production method of the present embodiment may be any powder that can be used as a raw material for recycled blanks. Preferably, the recycled powder is, for example, a recycled powder having an L* of 90 or greater and 100 or less, an a* of 0 or greater and 1 or less and a b* of 0 or greater and 5 or less (hereinafter, this powder is also referred to as the "present recycled powder").

The present recycled powder has an L* of 90 or greater and 100 or less, an a* of 0 or greater and 1 or less and a b* of 0 or greater and 5 or less and, preferably, has a lightness L*, a chromaticity a* and a chromaticity b*, according to the L*a*b* color system, that satisfy the following values.

Lightness L*: 90 or greater or 95 or greater and 100 or less or 99 or less,
Chromaticity a*: 0 or greater or 0.2 or greater and 1 or less or 0.8 or less, and
Chromaticity b*: 0 or greater or 0.2 or greater and 5 or less, 3 or less or 0.8 or less

In the case where the present recycled powder exhibits such a shade, the recycled powder can be used as the base powder. That is, the present recycled powder can be directly used in a method for producing a calcined body (dental blank). In addition, the present recycled powder may be a coloring-element-containing powder that can be used as the base powder. In the related art, a mixed powder system that uses, as the base powder, a powder containing no coloring element is used, and the calcined body (dental blank) suitable for dental prostheses is prepared by mixing a coloring-element-containing powder with the base powder. In contrast, in the case where the present recycled powder is used, horizontal recycling of the calcined body (dental blank) is made possible, and in addition, a mixed powder system including a coloring-element-containing powder as the base, which never existed before, can be realized. This mixed powder system enables the preparation of a calcined body (dental blank) suitable for dental prostheses, and by using the present recycled powder as the base powder, in addition to a powder containing no coloring element or in place of a powder not containing any coloring element at all, it is possible to obtain a dental prosthesis that exhibits any desired shade.

The shade of the recycled powder can be measured as follows. 3.0 g of the powder is loaded into a mold having a diameter of 25 mm and is uniaxially pressed at a pressure of 19.6 MPa. Then, the uniaxially pressed compact is subjected to a CIP process at a pressure of 196 MPa and formed to have a disc shape with a thickness of 3.0 ± 0.5 mm. A measurement surface thereof is polished with #800 waterproof abrasive paper to reduce the thickness by 0.1 mm and then is measured.

The present recycled powder is a powder prepared by using dental blanks as raw materials. Accordingly, the present recycled powder may be a powder containing zirconia as a major component and containing, as minor components, metal elements, such as a stabilizing element and a coloring element.

The stabilizing element that is included in the present recycled powder may be one or more selected from the group of yttrium, calcium and magnesium. Furthermore, the coloring element that is included in the present recycled powder may be one or more selected from the group of titanium, iron, cobalt, nickel, manganese, praseodymium, neodymium, europium, gadolinium, terbium, erbium and ytterbium. Preferably, the coloring element includes a transition metal element and a rare earth element; the transition metal element is one or more selected from the group of titanium, iron, cobalt, nickel and manganese, and the rare earth element is one or more selected from the group of praseodymium, neodymium, europium, gadolinium, terbium, erbium and ytterbium.

The present recycled powder is a powder that is obtainable by grinding a calcined body that has undergone molding and calcination. Since the present recycled powder has undergone these steps, it is believed that, in the present recycled powder, the coloring elements included in the zirconia include a coloring element that is in a state different from the state of a coloring element included as a result of immersion in a coloring solution or the state of a coloring element included as a result of the mixing of a solid phase. Accordingly, regarding the coloring elements of the present recycled powder, it is preferable that at least some of the coloring elements included be dissolved in the zirconia, and it is more preferable that at least some of the transition metal elements included be dissolved in the zirconia. Alternatively, the coloring elements may be included as different compounds (e.g., oxides).

### [Method for Producing Recycled Blank]

A method for producing a dental blank of the present embodiment may be any method that produces a recycled dental blank (recycled blank) by using a recycled powder produced by the production method of the present embodiment (hereinafter, this powder is also referred to as the "present recycled powder"). The method may be similar to known methods for producing a dental blank, with a difference being that the method uses the present recycled powder as a raw material powder. Furthermore, in the method for producing a recycled dental blank that uses the present recycled powder, the raw material powder to be used may be any powder including the present recycled powder, that is, the raw material powder to be used may be a mixed powder containing the recycled powder, for example, a mixed powder of the present recycled powder and a synthetic powder.

Specifically, the production method may be a method for producing a recycled dental blank including a molding step of molding the present recycled powder to form a green body; and a calcination step of calcining the green body.

The present recycled powder that is subjected to the molding step may be a recycled powder having a shade and a composition that have been appropriately adjusted in accordance with the shade of a desired dental prosthesis. For example, in a case where a dental blank (calcined body) for preparing a dental prosthesis having a shade corresponding to A1 of the Vita Classical shade guide is to be produced, the recycled powder can be used as the base powder. On the other hand, in a case where a dental blank (calcined body) for preparing a dental prosthesis having a shade darker than A1 of the Vita Classical shade guide is to be produced, the recycled powder can be used in a mixed powder of the base powder and a toning powder. In the mixed powder, the base powder serves as a dilution component with respect to the toning powder while the toning powder serves as a coloring element with respect to the base powder. Accordingly, in the case where a dental prosthesis having a darker shade is desired, this can be achieved by increasing the content ratio of the toning powder to the mixed powder.

Regarding molding methods for the molding step, any method that can mold the recycled powder into a green body (green compact) may be used. For example, the method may be one or more selected from the group of press molding, injection molding, sheet molding, extrusion molding and slip casting. The molding method may be any molding method suitable for the production of dental blanks. Preferably, the method is press molding.

The calcination step may be carried out by any calcination process (heat treatment) that enables the particles forming the recycled powder to become chemically adhered particles, and any calcination process for the production of known dental blanks may be used. Regarding conditions for the calcination, the process may be performed, for example, in an air atmosphere at a temperature of 800°C or greater and less than 1200°C.

The resulting recycled blank can be formed into a sintered body (dental prosthesis) by a known sintering method. The sintering method may be, for example, a sintering process in an air atmosphere at a temperature of 1200°C or greater and 1600°C or less.

### EXAMPLES

The present disclosure will now be described with reference to Examples. Note that the present disclosure is not limited to the Examples.

### <Composition Analysis>

The composition of composites was measured by ICP analysis. Sample powders were each subjected to a pretreatment for the analysis, which was a heat treatment performed in an air atmosphere at 1000°C for 1 hour.

### <Average Particle Size>

The average particle size of the powder samples was determined with a Planimetric method that uses an SEM image. Specifically, a circle having a known area was drawn on an SEM image, which was acquired under the following conditions, and the number of particles (Nc) of the ground product within the circle and the number of particles (Ni) of the ground product on the circumference of the circle were measured. The average particle size of the ground product was determined using equation (1), shown above, on the premise that the total number of particles (Nc+Ni) was 250 ± 50.

Acceleration magnification: 15 V
Magnification: 5000×

### <Density of Calcined Body>

A mass of the calcined body was determined by performing a measurement with an electronic balance, and a volume thereof was determined from dimensions measured with a caliper. A measured density was determined from the obtained mass and volume and taken as the density of the calcined body.

### <Density of Sintered Body>

The mass of the calcined body was determined by performing a measurement with an electronic balance, and a volume thereof was determined with an Archimedes method in accordance with JIS R 1634. A measured density was determined from the obtained mass and volume and taken as the density of the calcined body. The Archimedes method was performed by using ion-exchanged water as a solvent, and a pretreatment was performed with a boiling method.

### <Shade>

The shade was measured with a spectrophotometer (instrument name: CM-700d, manufactured by Konica Minolta Inc.) equipped with illumination and detection optics in accordance with Geometric condition c of JIS Z 8722; the measurement was performed by a method (black background measurement) in which a black calibration box was disposed as a background of the measurement sample. The measurement conditions were as follows.

Light source: D65 light source
Viewing angle: 10°
Measurement mode: SCI

The shade of the powder samples was measured as follows. 3.0 g of each of the powders was loaded into a mold having a diameter of 25 mm and was uniaxially pressed at a pressure of 19.6 MPa. Then, the uniaxially pressed compact was subjected to a CIP process at a pressure of 196 MPa and formed to have a disc shape with a thickness of 3.0 ± 0.5 mm. A measurement surface thereof was polished with #800 waterproof abrasive paper to reduce the thickness by 0.1 mm and then was measured.

The shade of the calcined body samples was determined by performing the measurement after polishing the measurement surface with #800 waterproof abrasive paper to reduce the thickness by 0.1 mm.

The sintered body samples were each measured after both surfaces of the sintered body were mirror-polished to have a thickness of 1.0 ± 0.1 mm and a surface roughness (Ra) of 0.02 µm or less.

### <Total Light Transmittance>

A total light transmittance was measured with a method compliant with JIS K 7361-1 by using a haze meter (instrument name: NDH4000, manufactured by Nippon Denshoku Industries Co., Ltd.) and a D65 light source. The measurement sample used was a disc-shaped sintered body having a thickness of 1.0 ± 0.1 mm, in which both surfaces had been polished to have a surface roughness Ra of ≤ 0.02 µm.

### Synthesis Example <Preparation of Calcined Body>

Calcined bodies having a composition and properties similar to those of dental blanks were prepared in the following manner to serve as simulated samples of scrap materials of dental blanks. Commercially available zirconia powders were added to a 200-mL propylene container in powder compounding ratios [mass%] shown in Tables 1 to 3 and were dry mixed by agitating the container (the powders were the following synthetic powders: for calcined bodies 1 to 4, Zpex, Zpex-Yellow, Zpex-Gray and Zpex-Pink; for calcined bodies 5 to 8, Zpex4, Zpex4-Yellow, Zpex-Gray and Zpex-Pink; and for calcined bodies 9 to 12, Zpex-Smile, Zpex-Smile-Yellow, ZpexSmile-Gray and Zpex-Pink, all manufactured by Tosoh Corporation). 26 g of the prepared powders were each loaded into a 57 mm × 34 mm mold and uniaxially pressed at a pressure of 19.6 MPa. Then, the uniaxially pressed compact was subjected to a CIP process at a pressure of 196 MPa to form a green body. The prepared green body was heat-treated under the following conditions to form a calcined body.

Calcination temperature: 1000°C
Calcination time: 1 hour
Heating rate: 50°C/hour
Calcination atmosphere: air atmosphere
Cooling rate: 300°C/hour

Similar operations were repeated to prepare calcined bodies 1 to 12, shown in Tables 1 to 3: for each of the calcined bodies, four samples were prepared. In the compositions of the calcined bodies shown in the tables below, the balance is zirconia.

**[Table 1]**

| | Powder Compounding Ratios [wt%] | | | | Composition of Calcined Body | | | | |
|---|---|---|---|---|---|---|---|---|---|
| | Zpex | Zpex-Yellow | Zpex-Gray | Zpex-Pink | Y [mol%] | Er [mol%] | Fe [wt%] | Co [wt%] | Al₂O₃ [wt%] |
| Calcined Body 1 | 68.39 | 30.00 | 1.61 | 0 | 2.883 | 0.049 | 0.045 | 0 | 0.050 |
| Calcined Body 2 | 68.92 | 30.00 | 1.08 | 0 | 2.877 | 0.033 | 0.045 | 0 | 0.050 |
| Calcined Body 3 | 62.25 | 30.00 | 1.08 | 6.67 | 2.878 | 0.033 | 0.045 | 0.003 | 0.050 |
| Calcined Body 4 | 31.18 | 60.00 | 2.15 | 6.67 | 2.849 | 0.066 | 0.090 | 0.003 | 0.050 |

**[Table 2]**

| | Powder Compounding Ratios [wt%] | | | | Composition of Calcined Body | | | | |
|---|---|---|---|---|---|---|---|---|---|
| | Zpex 4 | Zpex 4-Yellow | Zpex-Pink | Zpex-Gray | Y [mol%] | Er [mol%] | Fe [wt%] | Co [wt%] | Al₂O₃ [wt%] |
| Calcined Body 5 | 72.67 | 25.50 | 1.83 | 0 | 3.992 | 0.057 | 0.051 | 0 | 0.050 |
| Calcined Body 6 | 73.21 | 25.50 | 1.29 | 0 | 4.013 | 0.040 | 0.051 | 0 | 0.050 |
| Calcined Body 7 | 68.82 | 23.50 | 1.08 | 6.60 | 3.944 | 0.033 | 0.047 | 0.003 | 0.050 |
| Calcined Body 8 | 47.82 | 43.00 | 2.58 | 6.60 | 3.887 | 0.080 | 0.086 | 0.003 | 0.050 |

**[Table 3]**

| | Powder Compounding Ratios [wt%] | | | | Composition of Calcined Body | | | | |
|---|---|---|---|---|---|---|---|---|---|
| | Zpex Smile | Zpex Smile-Yellow | Zpex-Pink | Zpex Smile-Gray | Y [mol%] | Er [mol%] | Fe [wt%] | Co [wt%] | Al₂O₃ [wt%] |
| Calcined Body 9 | 73.85 | 24.00 | 2.15 | 0 | 5.190 | 0.067 | 0.048 | 0 | 0.050 |
| Calcined Body 10 | 74.39 | 24.00 | 1.61 | 0 | 5.218 | 0.050 | 0.048 | 0 | 0.050 |
| Calcined Body 11 | 72.43 | 20.00 | 1.00 | 6.49 | 5.245 | 0.034 | 0.040 | 0.003 | 0.050 |
| Calcined Body 12 | 58.28 | 32.00 | 3.23 | 6.49 | 5.135 | 0.101 | 0.064 | 0.003 | 0.050 |

### Examples 1 to 12 <Production of Recycled Powder>

The calcined bodies were ground in the following manner to provide a ground product. Specifically, four samples of calcined body 1 were prepared and coarsely ground in a zirconia mortar until they passed through a 1 mm-mesh sieve, to provide a coarsely ground product. The prepared coarsely ground product was ground in a ball mill using zirconia balls as grinding media. Accordingly, a ground product having an average particle size of 0.4 µm was obtained and used as a recycled powder of Example 1. A similar operation was performed on calcined bodies 2 to 12. Accordingly, ground products having an average particle size of 0.4 µm were obtained and used as respective recycled powders of Examples 2 to 12.

The evaluation results of the obtained recycled powders are shown in the table below.

**[Table 4]**

| | Raw Material | Shade of Recycled Powder | | | |
|---|---|---|---|---|---|
| | | L* | a* | b* | C* |
| Example 1 | Calcined Body 1 | 97.0 | 0.6 | 3.5 | 3.6 |
| Example 2 | Calcined Body 2 | 96.8 | 0.5 | 3.2 | 3.2 |
| Example 3 | Calcined Body 3 | 97.0 | 0.2 | 3.3 | 3.3 |
| Example 4 | Calcined Body 4 | 95.2 | 0.2 | 3.9 | 3.9 |
| Example 5 | Calcined Body 5 | 97.6 | 0.4 | 3.1 | 3.1 |
| Example 6 | Calcined Body 6 | 97.6 | 0.3 | 3.0 | 3.0 |
| Example 7 | Calcined Body 7 | 97.2 | 0.0 | 3.0 | 3.0 |
| Example 8 | Calcined Body 8 | 96.5 | 0.1 | 3.6 | 3.6 |
| Example 9 | Calcined Body 9 | 97.7 | 0.5 | 3.0 | 3.0 |
| Example 10 | Calcined Body 10 | 97.5 | 0.4 | 3.1 | 3.1 |
| Example 11 | Calcined Body 11 | 97.9 | 0.1 | 3.0 | 3.0 |
| Example 12 | Calcined Body 12 | 97.2 | 0.2 | 3.5 | 3.5 |

The obtained recycled powders had a lightness L* of 90 or greater and 100 or less (95.2 or greater and 97.9 or less), a chromaticity a* of 0 or greater and 1 or less (0.0 or greater and 0.6 or less), a chromaticity b* of 0 or greater and 5 or less (3.0 or greater and 3.9 or less) and a chroma C* of 3.0 or greater and 3.9 or less, and, therefore, all of the powders were recyclable powders that were able to serve as base powders.

### Examples 13 to 25 <Production of Recycled Blank>

3 g of each of the recycled powders of Examples 1 to 12 was loaded into a mold having a diameter of 25 mm and was uniaxially pressed at a pressure of 19.6 MPa. Then, the uniaxially pressed compact was subjected to a CIP process at a pressure of 196 MPa to form a green body. The prepared green bodies were heat-treated under the following conditions to form recycled blanks of Examples 13 to 25.

Calcination temperature: 1000°C
Calcination time: 1 hour
Heating rate: 50°C/hour
Calcination atmosphere: air atmosphere
Cooling rate: 300°C/hour

The evaluation results of the obtained recycled blanks are shown in the table below.

**[Table 5]**

| | Raw Material Powder | Shade | | | | Density of Calcined Body [g/cm³] |
|---|---|---|---|---|---|---|
| | | L* | a* | b* | C* | |
| Example 13 | Example 1 | 98.2 | 0.2 | 2.4 | 2.4 | 3.28 |
| Example 14 | Example 2 | 97.8 | 0.3 | 2.2 | 2.2 | 3.30 |
| Example 15 | Example 3 | 98.1 | 0.1 | 2.0 | 2.0 | 3.30 |
| Example 16 | Example 4 | 96.3 | 0.5 | 2.8 | 2.8 | 3.36 |
| Example 17 | Example 5 | 98.5 | 0.1 | 1.9 | 1.9 | 3.21 |
| Example 18 | Example 6 | 98.6 | 0.1 | 1.9 | 1.9 | 3.29 |
| Example 19 | Example 7 | 98.3 | 0.0 | 1.6 | 1.6 | 3.22 |
| Example 20 | Example 8 | 97.5 | 0.2 | 2.8 | 2.8 | 3.26 |
| Example 21 | Example 9 | 98.6 | 0.3 | 1.5 | 1.5 | 3.20 |
| Example 22 | Example 10 | 98.5 | 0.4 | 1.6 | 1.6 | 3.23 |
| Example 23 | Example 11 | 98.8 | 0.1 | 1.5 | 1.5 | 3.22 |
| Example 24 | Example 12 | 98.1 | 0.1 | 1.6 | 1.6 | 3.26 |

It was confirmed that the recycled powders produced by the method of the Examples are capable of being molded and calcined and are, therefore, usable as raw material powders for calcined bodies (recycled blanks). It was also confirmed that the obtained calcined bodies (recycled blanks) exhibit a shade comparable to that of recycled blanks prepared from a synthetic powder.

Furthermore, the obtained calcined bodies were sintered under the following conditions to form sintered bodies.

Sintering method: pressureless sintering
Sintering atmosphere: air atmosphere
Holding temperature: 1450°C (Examples 13 to 16 and 21 to 24)
   1500°C (Examples 17 to 20)
Holding time: 2 hours

The evaluation results of the obtained sintered bodies are shown in the table below, together with the evaluation results of sintered bodies obtained by sintering some of the calcined bodies obtained in the Synthesis Examples, under the same conditions. The shade designation indicates a color conforming to the Vita Classical shade guide.

**[Table 6]**

| Raw Material Powder | Shade | | | | Shade Designation | Total Light Transmittance [%] | Density of Calcined Body [g/cm³] |
|---|---|---|---|---|---|---|---|
| | L* | a* | b* | C* | | | |
| Example 13 | 78.1 | -2.3 | 9.2 | 9.5 | A1 | 36 | 6.09 |
| Example 14 | 78.2 | -2.5 | 9.7 | 10.0 | B1 | 36 | 6.09 |
| Example 15 | 76.2 | -1.3 | 8.2 | 8.3 | C1 | 31 | 6.09 |
| Example 16 | 75.4 | 0.4 | 14.1 | 14.1 | D2 | 26 | 6.09 |
| Example 17 | 76.7 | -1.8 | 8.8 | 9.0 | A1 | 40 | 6.08 |
| Example 18 | 77.0 | -2.3 | 8.7 | 9.0 | B1 | 41 | 6.08 |
| Example 19 | 74.7 | -1.2 | 7.0 | 7.1 | C1 | 36 | 6.08 |
| Example 20 | 72.2 | 0 | 12.2 | 12.2 | D2 | 32 | 6.08 |
| Example 21 | 73.6 | -2.5 | 8.4 | 8.8 | A1 | 44 | 6.05 |
| Example 22 | 74.5 | -2.3 | 9.1 | 9.4 | B1 | 44 | 6.05 |
| Example 23 | 72.5 | -2.1 | 5.5 | 5.9 | C1 | 42 | 6.05 |
| Example 24 | 70.3 | -0.9 | 10.2 | 10.2 | D2 | 39 | 6.06 |
| Calcined Body 1 | 78.3 | -2.5 | 9.5 | 9.8 | A1 | 36 | 6.09 |
| Calcined Body 2 | 77.1 | -2.2 | 9.0 | 9.3 | A1 | 40 | 6.08 |
| Calcined Body 3 | 73.9 | -2.1 | 8.7 | 8.9 | A1 | 44 | 6.05 |

As indicated in the table above, it was confirmed that sintered bodies having aesthetic properties suitable for dental prostheses can be obtained from the recycled blanks obtained in the Examples. Furthermore, comparisons between Example 13 and calcined body 1, between Example 17 and calcined body 5 and between Example 21 and calcined body 9 indicate that the dental prostheses obtained from the recycled blanks of the Examples had the same translucency and the same density of the sintered body and exhibited similar shades, compared to the dental blanks prepared from synthetic powders. Thus, it was confirmed that the sintered bodies (dental prostheses) obtained from the recycled powders are sintered bodies comparable to sintered bodies (dental prostheses) prepared from synthetic powders.

### Example 25 <Production of Recycled Blank from Mixed Powder>

Mixed powders were prepared in a manner similar to that of Synthesis Example 1, except that the recycled powders obtained in Examples 5 to 8 and synthetic powders (product names: Zpex 4-Yellow, Zpex-Gray and Zpex-Pink, all manufactured by Tosoh Corporation) that served as toning powders were used, and that the compounding ratios shown in the table below were employed.

**[Table 7]**

| | Powder Compounding Ratios | | | | |
|---|---|---|---|---|---|
| | Recycled Powder | | Zpex 4-Yellow [wt%] | Zpex-Pink [wt%] | Zpex-Gray [wt%] |
| | Type | Compounding Ratio [wt%] | | | |
| Example 25 | Example 5 | 31.7 | 56.0 | 5.2 | 7.1 |
| Example 26 | Example 6 | 62.8 | 36.0 | 1.2 | 0.0 |
| Example 27 | Example 7 | 28.5 | 57.3 | 0.8 | 13.4 |
| Example 28 | Example 8 | 86.2 | 12.8 | 1.0 | 0.0 |

Recycled blanks were obtained in a manner similar to that of Examples 13 to 24, except that the obtained mixed powders were used. The evaluation results of the obtained recycled blanks are shown in the table below.

**[Table 8]**

| | Shade | | | | Shade Designation | Density of Calcined Body [g/cm³] |
|---|---|---|---|---|---|---|
| | L* | a* | b* | C* | | |
| Example 25 | 95.9 | 0.8 | 4.8 | 4.9 | A4 | 3.30 |
| Example 26 | 96.9 | 0.5 | 3.9 | 3.9 | B4 | 3.27 |
| Example 27 | 95.9 | 0.6 | 4.4 | 4.4 | C4 | 3.30 |
| Example 28 | 97.1 | 0.4 | 3.2 | 3.2 | D3 | 3.26 |

These results confirm that the recycled powders of the Examples are mixed powders that are usable as raw materials for recycled blanks. Sintered bodies were obtained by sintering the obtained calcined bodies (recycled blanks). The sintering was performed in a manner similar to that of Examples 13 to 24, except that the obtained calcined bodies were used.

The results are shown in the table below.

**[Table 9]**

| | Shade | | | | Shade Designation | Total Light Transmittance [%] | Density of Calcined Body [g/cm³] |
|---|---|---|---|---|---|---|---|
| | L* | a* | b* | C* | | | |
| Example 25 | 69.8 | 2.5 | 15.1 | 15.3 | A4 | 28 | 6.09 |
| Example 26 | 74.1 | -0.7 | 15.6 | 15.6 | B4 | 34 | 6.08 |
| Example 27 | 68.6 | 1.5 | 13.9 | 14.0 | C4 | 26 | 6.08 |
| Example 28 | 71.7 | 0.5 | 13.3 | 13.3 | D4 | 33 | 6.08 |

As indicated in the table above, it was confirmed that in the case where any of the recycled powders of the Examples is mixed with a toning powder to form a mixed powder, a sintered body having aesthetic properties suitable for dental prostheses having a darker shade can be obtained, and that the recycled powders are usable as recycled raw material for that purpose. In addition, it was confirmed that either in the case where a coloring-element-containing zirconia powder obtained from any of the recycled powders of the Examples is used as a base powder or in the case where a mixed powder system obtained from any of the recycled powders is used, a sintered body (dental prosthesis) that exhibits any suitable shade for a dental prosthesis can be obtained.

The entire contents of the specifications, claims, drawings and abstracts of Japanese Patent Application No. 2023-201429, filed on November 29, 2023, and Japanese Patent Application No. 2024-006646, filed on January 19, 2024, are cited and incorporated herein as a disclosure of the specification of the present disclosure.

## Claims

1. A method for producing a recycled powder, the method comprising:
a grinding step of grinding scrap materials of dental blanks to provide a ground product; and
a collection step of collecting, from the ground product, a powder having a shade that has an L* of 90 or greater and 100 or less, an a* of 0 or greater and 1 or less and a b* of 0 or greater and 5 or less.

2. The method for producing a recycled powder according to claim 1, further comprising a separation step of separating the scrap materials prior to the grinding step.

3. The method for producing a recycled powder according to claim 2, wherein the separation step includes separating the scrap materials into a first shade unit and a second shade unit, the first shade unit is formed of at least one scrap material having a base shade, and the second shade unit is formed of at least one scrap material having a shade different from the base shade.

4. The method for producing a recycled powder according to any one of claims 1 to 3, wherein the grinding step includes a coarse grinding step and a particle size adjustment step, the coarse grinding step is a step of dry-grinding the scrap materials to provide a coarsely ground product, and the particle size adjustment step is a step of wet-grinding the coarsely ground product.

5. The method for producing a recycled powder according to any one of claims 1 to 4, wherein the collection step includes separating the ground product provided in the grinding step into several units and then collecting, from the several units, a unit corresponding to a portion of the ground product, the portion having a shade that has an L* of 90 or greater and 100 or less, an a* of 0 or greater and 1 or less and a b* of 0 or greater and 5 or less.

6. A method for recycling dental blanks, the method comprising:
a grinding step of grinding scrap materials of the dental blanks to provide a ground product; and
a collection step of collecting, from the ground product, a powder having a shade that has an L* of 90 or greater and 100 or less, an a* of 0 or greater and 1 or less and a b* of 0 or greater and 5 or less.

7. A method for producing a recycled dental blank, the method comprising using the recycled powder obtainable according to claim 1 or 2.

8. A recycled powder having a shade that has an L* of 90 or greater and 100 or less, an a* of 0 or greater and 1 or less and a b* of 0 or greater and 5 or less.
